(19) Europäisches Patentamt
European Patent Office
Office européen des brevets

(11) **EP 2 484 686 A1**

(12) **EUROPEAN PATENT APPLICATION**
published in accordance with Art. 153(4) EPC

(43) Date of publication:
**08.08.2012 Bulletin 2012/32**

(21) Application number: **10820475.1**

(22) Date of filing: **27.09.2010**

(51) Int Cl.:
*C07H 7/027* [(2006.01)]  *A23C 9/142* [(2006.01)]
*A23C 9/152* [(2006.01)]  *A23L 1/30* [(2006.01)]
*B01D 61/14* [(2006.01)]

(86) International application number:
**PCT/JP2010/066669**

(87) International publication number:
**WO 2011/040360 (07.04.2011 Gazette 2011/14)**

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **29.09.2009 JP 2009223848**

(71) Applicant: **Megmilk Snow Brand Co., Ltd.**
**Sapporo-shi**
**Hokkaido**
**0650043 (JP)**

(72) Inventors:
• **SOGAME, Hitomi**
  **Sapporo-shi**
  **Hokkaido 065-0043 (JP)**

• **ITOH, Kotaro**
  **Sapporo-shi**
  **Hokkaido 065-0043 (JP)**
• **TOMIZAWA, Akira**
  **Sapporo-shi**
  **Hokkaido 065-0043 (JP)**
• **YAMAZAKI, Ryu**
  **Sapporo-shi**
  **Hokkaido 065-0043 (JP)**
• **YAMADA, Hiroaki**
  **Sapporo-shi**
  **Hokkaido 065-0043 (JP)**

(74) Representative: **MacLean, Martin Robert**
**Mathys & Squire LLP**
**120 Holborn**
**London**
**EC1N 2SQ (GB)**

(54) **METHOD FOR SEPARATING SIALYLLACTOSE MATERIAL**

(57)     A method for easily and efficiently separating a sialyllactose material from a milk material is disclosed. The method comprises removing proteins from the milk material to obtain a protein-free liquid, adjusting the pH of the protein-free liquid to obtain a pH-adjusted liquid, and concentrating the pH-adjusted liquid by an ultrafiltration (UF) membrane. Since the method also allows separation of sialyllactose from lactose and minerals, sialyllactose can be obtained with high purity, making the sialyllactose material highly useful for food, pharmaceutical, and other applications.

EP 2 484 686 A1

**Description**

**TECHNICAL FIELD**

The present invention relates to a method of separating a sialyllactose material from milk (i.e., starting material), comprising removing proteins from a milk material to obtain a protein-free liquid, adjusting the pH of the protein-free liquid to obtain a pH-adjusted liquid, and concentrating the pH-adjusted liquid by an ultrafiltration (UF) membrane. The invention also relates to food, beverages and the like that comprise the resulting sialyllactose material.

**BACKGROUND ART**

Sialyllactose, in which a sialic acid is bonded to a lactose, is known to have a protective effect against virus and bacteria infections and exhibit various physiological activities such as a growth-promoting activity for lactic acid bacteria. Therefore, sialyllactose has been used in formula milk for infants and the like (see Non-patent Document 1, for example). Sialyllactose is also known to have inhibitory effects on HIV infection/proliferation, and therefore its application in food products designed for inhibiting HIV infection/proliferation is also expected (see Patent Document 1, for example).

A method that separates and collects sialic acid compounds from milk or whey on an industrial scale by using a simulated moving bed (SMB) chromatographic separator (see Patent Document 2, for example), and a method that separates sialic acid compounds from milk or whey by using an ion-exchange resin (see Patent Document 3, for example) have been disclosed.

A method that separates a glycomacropeptide, which is a sialic acid-binding peptide, via fractionation using a membrane having a molecular weight cutoff of 1 kDa or higher has also been disclosed (see Patent Document 4, for example).

**RELATED-ART DOCUMENTS**

**PATENT DOCUMENTS**

Patent Document 1: JP-A-2006-117597
Patent Document 2: JP-A-H08-252403
Patent Document 3: JP-A-H11-180993
Patent Document 4: JP-A-2003-246800

**NON-PATENT DOCUMENT**

Non-patent Document 1: Shokuhin To Kaihatsu, Vol. 30, pp. 10-13

**SUMMARY OF THE INVENTION**

**PROBLEMS TO BE SOLVED BY THE INVENTION**

The method that uses a simulated moving bed (SMB) chromatographic separator (Patent Document 2) could become problematic due to the overly complicated operation conditions when simultaneously purifying sialic acid compounds having different separation factors, for example. Moreover, the method of Patent Document 2 is not suitable for a mass production purpose since large equipments would be needed to obtain a large amount of sialic acid compounds. The method that uses an ion-exchange resin (Patent Document 3) has difficulty when used on a large scale due to the poor chemical resistance and physical durability of the resin and the environmental pollution caused by the regeneration effluent, for example.

The present inventors conducted extensive research seeking a method that can more easily separate a sialyllactose material using equipments and operating conditions that are more suited for mass production operations. As a result, the inventors found that sialyllactose can be easily and efficiently separated from lactose and minerals contained in a milk material, by removing proteins from the milk material, adjusting a pH of the resulting liquid, and concentrating the pH-adjusted liquid by using a membrane. The invention uses an ultrafiltration (UF) membrane for concentrating sialyllactose in the liquid from which proteins have been removed. Thus, a flux can be maintained at a relatively low pressure compared to a nanofiltration (NF) membrane, and less energy is required for the operation. Moreover, the environmental impact of the effluent water generated during the washing step of the membrane is significantly lower than that of the regeneration effluent water generated with the use of the resin. The above findings have led to the completion of the invention. An object of the invention is to provide a method that can easily and efficiently separate a sialyllactose material from a milk material, and to provide food, beverages and the like that comprise the resulting sialyllactose material.

**MEANS OF SOLVING THE PROBLEMS**

The present invention comprises the following:

(1) A method of separating a sialyllactose material, comprising removing proteins from a milk material to obtain a protein-free liquid, adjusting a pH of the protein-free liquid to obtain a pH-adjusted liquid, and concentrating the pH-adjusted liquid by an ultrafiltration (UF) membrane.
(2) The method according to (1), wherein proteins are removed from the milk material by an ultrafiltration (UF) membrane.
(3) The method according to (1) or (2), wherein proteins are removed from the milk material by an ultrafiltration (UF) membrane having a molecular weight cutoff of 5 to 16 kDa, and the pH-adjusted liquid is concentrated by an ultrafiltration (UF) membrane having a molecular weight cutoff of 600 Da to 3 kDa.
(4) The method according to any one of (1) to (3), wherein skim milk is used as the milk material, and the pH of the protein-free liquid is adjusted to 5.6 to 6.6.
(5) The method according to any one of (1) to (3), wherein whey is used as the milk material, and the pH of the protein-free liquid is adjusted to 3.6 to 5.5.
(6) A sialyllactose material obtainable by a process comprising removing proteins from a milk material to obtain a protein-free liquid, adjusting a pH of the protein-free liquid to obtain a pH-adjusted liquid, and concentrating the pH-adjusted liquid by an ultrafiltration (UF) membrane.
(7) A processed food comprising the sialyllactose material according to (6).
(8) A processed food comprising 0.1 to 90 g of the sialyllactose material according to (6) per 100 g of the processed food.
(9) A formula milk comprising the sialyllactose material according to (6).
(10) A formula milk comprising 0.1 to 30 g of the sialyllactose material according to (6) per 100 g of the formula milk.

**EFFECTS OF THE INVENTION**

The method of the present invention can separate a high-purity sialyllactose material from a milk material (i.e., starting material) through simple operations.

**DESCRIPTION OF EXEMPLARY EMBODIMENTS**

Examples of the milk material used as the starting material include skim milk, cheese whey, acid whey, rennet whey, butter milk, and the like that are obtained from the milk of a mammal such as cow milk, goat milk, sheep milk, camel milk, and horse milk. Since the proteins contained in these milk materials would decrease the efficiency of the subsequent membrane-concentration step, it is necessary to remove proteins from the milk materials.
Proteins may be removed from the milk material by any methods that are capable of removing proteins, including membrane treatment, adsorption treatment, aggregation treatment, and the like.
A membrane treatment may be performed by using an ultrafiltration (UF) membrane. In particular, an ultrafiltration membrane having a molecular weight cutoff of 5 to 16 kDa can efficiently remove proteins (particularly whey proteins), and an ultrafiltration membrane having a molecular weight cutoff of 10 kDa can more efficiently separate the sialyllactose material. Specific examples of the ultrafiltration (UF) membranes include HFK131 (manufactured by KOCH Membrane System Inc.), PW (manufactured by GE Osmonics), and the like.
An adsorption treatment may be performed by using an adsorbent. Proteins can be removed from the milk material by bringing the adsorbent into contact with the milk material to allow the proteins to be adsorbed on the adsorbent. Specific examples of the adsorbents include ion-exchange resins such as QMA Spherosil and S Spheroil (both manufactured by Pall Corporation).
In an aggregation treatment, proteins may be subjected to aggregation by adjusting the pH of the milk material and/or by heating the milk material, and the aggregated proteins may be removed by solid-liquid separation. Caseins that constitute about 80% of the proteins in the milk material can be aggregated by adjusting the pH of the milk material to 4.6, and whey proteins that constitute about 20% of the proteins in the milk material can be aggregated by adjusting the pH of the milk material to 3.5 to 5.5 and/or by heating the milk material to 55°C or higher. An acid or a base may be used to adjust the pH of the milk material. Specific examples of the acids and the bases include hydrochloric acid, lactic acid, caustic soda, and the like. The aggregated proteins may be removed by solid-liquid separation methods such as sedimentation (e.g., sedimentation using the "Separator MSD" system (manufactured by GEA Westfalia Separator)), compression (e.g., compression using the "Clarifying Decanter CA" system (manufactured by GEA Westfalia Separator)), and the like.

At least about 80% (preferably almost 100%) of the proteins can be removed from the milk material through any of these treatments to obtain a protein-free liquid. The concentrated protein solution, a by-product produced during the protein removal step, contains a large amount of proteins, and thus may be used as a raw material for producing protein materials such as MPC and WPC.

In the present invention, it is very important to adjust the pH of the protein-free liquid. The main component of the solutes contained in the protein-free liquid is lactose. Therefore, it is important to separate sialyllactose from lactose if sialyllactose is to be separated from the protein-free liquid. The inventors carefully studied the behaviors of sialyllactose and lactose during the ultrafiltration membrane treatment of the protein-free liquid. As a result, the inventors found that the sialyllactose rejection rate on an ultrafiltration membrane depends on the pH of the protein-free liquid. Specifically, the sialyllactose rejection rate on an ultrafiltration membrane is higher when the pH of the protein-free liquid is on the more acidic side, and lower when the pH of the protein-free liquid is on the more neutral side. The inventors also found that the lactose rejection rate on an ultrafiltration membrane similarly depends on the pH of the protein-free liquid. The term "rejection rate" used herein refers to the percentage of a specific component (component A, for example) that is retained by the membrane and thus cannot pass through the membrane during membrane separation. The rejection rate may be expressed by the following formula:

$$\text{Component A rejection rate (\%)} = (1 - (\text{concentration of component A in the liquid that has passed through membrane} / \text{concentration of component A in the concentrate})) \times 100$$

It is particularly worth noting that the sialyllactose rejection rate is high while the lactose rejection rate is low in a specific pH region, which results in a large difference between the rejection rates of the two components. Due to this difference in the rejection rates, sialyllactose can be separated from lactose, and thus sialyllactose can be concentrated from the protein-free liquid. The inventors also conducted extensive studies on a plurality of protein-free liquids obtained from various milk materials. As a result, the inventors found a specific pH region for each milk material in which sialyllactose is efficiently separated from lactose. Specifically, if skim milk is used as the milk material (i.e., starting material), a preferable pH is 5.6 to 6.6, and a more preferable pH is 5.6 to 6.3. If whey is used as the milk material, the pH is preferably 3.6 to 5.5, more preferably 3.6 to 5.0, and particularly preferably 3.6 to 4.5.

The pH of the protein-free liquid is adjusted by using an acidic solution or a basic solution depending on the pH of the protein-free liquid. The types of the acidic solution and the basic solution are not particularly limited. Examples of the acidic solution and the basic solution include hydrochloric acid and sodium hydroxide solutions. The pH of the protein-free liquid may also be adjusted by using an ion-exchange resin in order to minimize an increase in the amount of minerals. However, if the pH is too high, calcium phosphate may precipitate, which would foul the membrane and decrease the flow rate in the subsequent step. Therefore, it is preferable that the pH of the protein-free liquid be set within the above-mentioned ranges.

The pH-adjusted liquid thus obtained is then concentrated by an ultrafiltration (UF) membrane. The pH-adjusted liquid may preferably be concentrated by a membrane having a molecular weight cutoff of 600 Da to 3 kDa, and more preferably a molecular weight cutoff of 1 kDa. Specific examples of the membranes that could be used in the present invention include Membralox 1kDa (manufactured by Pall Exekia) and the XT membrane (manufactured by Synder Filtration). When the pH-adjusted liquid whose pH has been appropriately adjusted is concentrated by an ultrafiltration (UF) membrane having a molecular weight cutoff within the above-mentioned ranges, sialyllactose will be retained (excluded) by the ultrafiltration membrane at a high rate and remain in the concentrate fraction. On the other hand, since lactose is retained by the ultrafiltration membrane at a lower rate, most of the lactose will pass through the ultrafiltration membrane to collect in the permeate (pass-through) fraction. Sialyllactose can thus be separated.

If a diafiltration (DF) treatment is performed, in which the sialyllactose-containing concentrate liquid obtained by the ultrafiltration (UF) membrane is further concentrated while water is added to the liquid, certain amounts of lactose and minerals can be further removed from the said concentrate liquid, thereby increasing the sialyllactose concentration. Since the method according to the invention can also separate sialyllactose from lactose and minerals, it is possible to obtain a high-purity sialyllactose material. Therefore, the sialyllactose material obtained by the method according to the invention is highly useful for food, pharmaceutical, and other applications.

The sialyllactose material obtained as above may be incorporated in processed foods, formula milk and similar food products, medical drugs, animal feed, and the like.

Examples of the processed foods include those comprising sugar, glucose, malt sugar, cornstarch, dextrin, sugar alcohol, lactose, or the like as a main ingredient. The processed food may contain 0.1 to 90 g of the sialyllactose material per 100 g of the product. The processed food may take any forms such as solid, semi-solid, and liquid, and it may also be in a tablet form. In the above, the lower limit of the sialyllactose material content is said to be 0.1 g per 100 g of the processed food; this is because this concentration is equivalent to the sialyllactose content of the human milk (breast milk) (in terms of solid mass), and if the processed food contains less than 0.1 g of the sialyllactose material per 100 g, the presence of the sialyllactose may be less meaningful, or no significant effect of the sialyllactose may be obtained. Examples of the formula milk include formula milk for infants, formula milk for low-birth-weight infants, follow-up milk, formula milk for infants having allergy diseases, and the like. The formula milk may contain 0.1 to 30 g of the sialyllactose material per 100 g of the product. This is because a predominant component of the sialyllactose material is lactose and therefore addition of more than 30 g of the sialyllactose material per 100 g of the product is difficult given the compositional requirements imposed on the formula milk. The lower limit of the sialyllactose material content is said to be 0.1 g per 100 g of the formula milk because this concentration is equivalent to the sialyllactose content of the human milk (breast milk) (in terms of solid mass). If the formula milk contains less than 0.1 g of the sialyllactose material per 100 g, the presence of the sialyllactose may be less meaningful, or no significant effect of the sialyllactose may be obtained.

The invention is further described below by examples. The following examples are given for illustrative purposes, and should not be construed as limiting the invention.

[Example 1]

Considerations on protein removal by membranes

The effect of the pore size of the membrane used for removing proteins from the starting material was investigated.

Skim milk was used as the milk material (i.e., starting material). Proteins were removed from the skim milk by using a UF membrane having a molecular weight cutoff of 10 kDa (HFK131 manufactured by KOCH Membrane System Inc.) or a UF membrane having a molecular weight cutoff of 5 kDa (Membralox 5 kDa manufactured by Pall Exekia) to obtain a protein-free liquid. The pH of the protein-free liquid was adjusted to 6.3, 5.6, or 4.5 by using 1N hydrochloric acid to obtain a pH-adjusted liquid, and the pH-adjusted liquid was treated at 50°C with a UF membrane having a molecular weight cutoff of 1 kDa (Membralox 1 kDa manufactured by Pall Exekia). The sialyllactose concentrations in the concentrate fraction and in the permeate fraction that passed through the UF membrane were measured by high-performance liquid chromatography (HPLC). An HPLC system "DX500" (manufactured by DIONEX) and a "CarboPac PA1" column were used for the measurement. A 60 mM sodium acetate/100 mM sodium hydroxide solution was used as the mobile phase, and the flow rate was 1 ml/min.

The sialyllactose rejection rates were calculated from the sialyllactose concentrations thus measured.

The evaluation results are shown in Table 1. When skim milk that had not been subjected to protein removal was treated with the UF membrane having a molecular weight cutoff of 1 kDa, the proteins fouled the surface of the membrane immediately after the start of the operation, and the operation could not be continued. On the other hand, when the protein-free liquid obtained by using a UF membrane having a molecular weight cutoff of 10 kDa or 5 kDa was treated with a UF membrane having a molecular weight cutoff of 1 kDa, the membrane fouling was not observed, and the membrane separation operation could be stably continued. This indicates that the step of removing proteins is essential in the present invention. The same results were obtained when other milk materials (i.e., starting materials) were used.

When proteins were removed by using the UF membrane having a molecular weight cutoff of 10 kDa, the sialyllactose rejection rate on the UF membrane having a molecular weight cutoff of 1 kDa was 100% at all pH values tested between 6.3 and 4.5. On the other hand, when proteins were removed by using the UF membrane having a molecular weight cutoff of 5 kDa, the sialyllactose rejection rate on the UF membrane having a molecular weight cutoff of 1 kDa was 77.4% and 42.2% at the pH of 5.6 and 6.3, respectively, indicating that sialyllactose was leaking into the permeate fraction. If sialyllactose leaks into the permeate fraction, the yield of sialyllactose would decrease, and the production cost would increase. It was thus found that a UF membrane having a molecular weight cutoff of 10 kDa is more preferably used for the removal of the proteins.

Since 95% or more of the proteins contained in the milk material have a molecular weight of higher than 16 kDa, a UF membrane having a molecular weight cutoff of 16 kDa or lower needs to be used for the removal of the proteins. Use of a UF membrane having a molecular weight cutoff of lower than 5 kDa would result in a reduced sialyllactose rejection rate and a reduced removal efficiency. Thus, for the step of removing proteins, it is more preferable to use a UF membrane having a molecular weight cutoff of 5 to 16 kDa.

[TABLE 1]

| Molecular weight cutoff of the membrane used for the protein removal | Adjusted pH | Sialyllactose exclusion rate (%) on a 1 kDa cutoff UF membrane |
|---|---|---|
| No removal | Not adjusted | Not measurable (membrane clogging) |
| 10kDa | 6.3 | 100 |
| | 5.6 | 100 |
| | 4.5 | 100 |
| 5kDa | 6.3 | 42.2 |
| | 5.6 | 77.4 |
| | 4.5 | 100 |

[Example 2]

Optimum pH when cheese whey is used as starting material

The treatment conditions when cheese whey is used as the milk material (i.e., starting material) were investigated.

Proteins were removed from cheese whey at 10°C by using a UF membrane having a molecular weight cutoff of 10 kDa ("HFK131" manufactured by KOCH Membrane System Inc.) to obtain a protein-free liquid. The pH of the protein-free liquid was appropriately adjusted by using 1N hydrochloric acid to obtain a pH-adjusted liquid, and the pH-adjusted liquid was treated (total circulation) at 50°C with a UF membrane having a molecular weight cutoff of 1 kDa. The sialyllactose concentration and the lactose concentration in the resulting permeate fraction were measured, and the rejection rates were each evaluated. The sialyllactose concentration was measured in the same manner as in Example 1, and the lactose concentration was measured by using a Brix meter (manufactured by Atago Co., Ltd.).

The evaluation results are shown in Table 2. When whey is used as the starting material, sialyllactose could be most efficiently concentrated if the pH of the treatment has been adjusted to 3.6 to 5.5, particularly 3.6 to 5.0, due to the increased difference between the sialyllactose and lactose rejection rates.

[TABLE 2]

| pH during the treatment with a 1kDa cutoff membrane | Sialyllactose exclusion rate (%) | Lactose exclusion rate (%) |
|---|---|---|
| 6.0 | 45.9 | 13.5 |
| 5.5 | 51.5 | 10.0 |
| 5.0 | 49.1 | 15.4 |
| 4.5 | 62.4 | 21.1 |
| 4.0 | 96.7 | 47.1 |
| 3.6 | 93.0 | 51.0 |

[Example 3]

Optimum pH when skim milk is used as starting material

The treatment conditions when skim milk is used as the milk material (i.e., starting material) were investigated.

Proteins were removed from skim milk at 10°C by using a UF membrane having a molecular weight cutoff of 10 kDa ("HFK131" manufactured by KOCH Membrane System Inc.) to obtain a protein-free liquid. The pH of the protein-free liquid was appropriately adjusted by using 1N hydrochloric acid to obtain a pH-adjusted liquid, and the pH-adjusted liquid was treated (total circulation) at 50°C with a UF membrane having a molecular weight cutoff of 1 kDa. The sialyllactose concentration and the lactose concentration in the resulting permeate fraction were measured, and the rejection rates were each evaluated. The sialyllactose concentration was measured in the same manner as in Example 1, and the lactose concentration was measured in the same manner as in Example 2.

The evaluation results are shown in Table 3. As is clear from the results shown in Table 3, when skim milk is used as the starting material, sialyllactose could be most efficiently concentrated if the pH of the treatment has been adjusted to 5.6 to 6.6, particularly 5.6 to 6.3.

[TABLE 3]

| pH during the treatment with a 1kDa cutoff membrane | Sialyllactose exclusion rate (%) | Lactose exclusion rate (%) |
|---|---|---|
| 6.9 | 43.2 | 7.4 |
| 6.6 | 65.1 | 12.3 |
| 6.3 | 100 | 26.9 |
| 5.6 | 100 | 44.2 |
| 4.5 | 100 | 78.2 |

[Example 4]

Separation of a sialyllactose material from a cheese whey starting material

610 kg of cheese whey was concentrated to volume concentration factor 5 at 10°C by using a UF membrane having a molecular weight cutoff of 10 kDa ("PW3838-50D" manufactured by GE Osmonics) to obtain 488 kg of a protein-free liquid which was depleted of proteins. The pH of this protein-free liquid was adjusted to 5.0 by using 1N hydrochloric acid to obtain a pH-adjusted liquid. The pH-adjusted liquid was concentrated to volume concentration factor 44 at 50°C by using a UF membrane having a molecular weight cutoff of 1 kDa (Membralox 1 kDa manufactured by Pall Exekia), and then subjected to a 0.9-fold diafiltration (DF) treatment to obtain a sialyllactose concentrate. The composition of the sialyllactose-enriched material obtained by freeze-drying the said sialyllactose concentrate is shown in Table 4. The sialyllactose content in this material was 0.65% based on the solid weights.

[TABLE 4]

|  | Cheese whey | Sialyllactose-enriched material |
|---|---|---|
| Solids [g/100g] | 6 | 98 |
| Sialyllactose [mg/100g] | 5. 4 | 636 |
| Sialyllactose per solids [%] | 0. 09 | 0. 65 |

[Example 5]

Separation of a sialyllactose material from a skim milk starting material

700 kg of raw milk was separated by using a milk separator to obtain 605 kg of skim milk. The skim milk was pasteurized by heating it to 75°C, and concentrated to volume concentration factor 4 at 50°C by using a UF membrane having a molecular weight cutoff of 10 kDa ("DESAL PW3838-30D" manufactured by GE Osmonics) to obtain 450 kg of a protein-free liquid which was depleted of proteins. The pH of the protein-free liquid was adjusted to 6.3 to obtain a pH-adjusted liquid The pH-adjusted liquid was concentrated to volume concentration factor 36 by using a UF membrane having a molecular weight cutoff of 1 kDa ("Membralox 1 kDa" manufactured by Pall Exekia), and then subjected to a 1.1-fold diafiltration (DF) treatment to obtain a sialyllactose-enriched material. The composition of the freeze-dried sialyllactose-enriched material is shown in Table 5. The sialyllactose content in this material was 0.95% based on the solid weights.

[TABLE 5]

|  | Skim milk | Sialyllactose-enriched material |
|---|---|---|
| Solids [g/100g] | 9 | 9 7 |
| Sialyllactose [mg/100g] | 6. 1 | 919 |
| Sialyllactose per solids [%] | 0. 07 | 0. 95 |

[Example 6]

Whey powder, powdered skim milk, concentrated-protein whey powder, butter milk, and whole milk powder (all manufactured by Snow Brand Milk Products Co., Ltd.) were mixed in the amounts shown in Table 6 to obtain a solution. Casein (manufactured by Fonterra Ltd.), a milk serum protein concentrate (manufactured by Fonterra Ltd.), vitamins (manufactured by BASF), and minerals (manufactured by Komatsuya Corporation) were added to the solution, and further, the sialyllactose material produced in Example 5 was added, and other minor components were added. After the addition of an oil and fat mixture (manufactured by Ueda Oils And Fats Mfg Co., Ltd.), the mixture was mixed, pasteurized, and spray-dried, to obtain sialyllactose-fortified formula milk for infants.
Since the resulting formula milk is fortified with sialyllactose, the formula milk provides protective effects against viral and bacterial infections, as well as effects of facilitating various physiological activities such as a growth-promoting activity for lactic acid bacteria.
[TABLE 6]

| Whey powder, powdered skim milk, concentrated-protein whey powder, butter milk, whole milk powder | 53.8 (%) |
|---|---|
| Casein | 2.0 |
| Milk serum protein concentrate | 1.0 |
| Oil and fat mixture | 27.0 |
| Sialyllactose material | 15.0 |
| Minerals | 1.0 |
| Vitamins | 0.1 |
| Other minor components | 0.1 |

[Example 7]

Glucose (manufactured by San-ei Sucrochemical Co., Ltd.), a sucrose ester (manufactured by Mitsubishi-Kagaku Foods Corporation), crystalline cellulose (manufactured by Asahi Kasei Chemicals Corporation), and flavors (manufactured by Takasago International Corporation) were mixed in the amounts shown in Table 7. The sialyllactose material produced in Example 5 was added to the mixture to obtain a mixed powder. The mixed powder was directly formed into tablets at a pressure of 1 to 3 t to obtain 1 g of sialyllactose-containing tablets.
Since the resulting tablet is fortified with sialyllactose, it provides protective effects against viral and bacterial infections, as well as effects of facilitating various physiological activities such as a growth-promoting activity for lactic acid bacteria.
[TABLE 7]

| Glucose | 53.0(%) |
|---|---|
| Sialyllactose material | 40.0 |
| Sucrose ester | 1.0 |
| Crystalline cellulose | 5.0 |
| Flavors | 1.0 |

Claims

1.   A method of separating a sialyllactose material, comprising removing proteins from a milk material to obtain a protein-free liquid, adjusting a pH of the protein-free liquid to obtain a pH-adjusted liquid, and concentrating the pH-adjusted liquid by an ultrafiltration (UF) membrane.

2. The method according to claim 1, wherein proteins are removed from the milk material by an ultrafiltration (UF) membrane.

3. The method according to claim 1 or 2, wherein proteins are removed from the milk material by an ultrafiltration (UF) membrane having a molecular weight cutoff of 5 to 16 kDa, and the pH-adjusted liquid is concentrated by an ultrafiltration (UF) membrane having a molecular weight cutoff of 600 Da to 3 kDa.

4. The method according to any one of claims 1 to 3, wherein skim milk is used as the milk material, and the pH of the protein-free liquid is adjusted to 5.6 to 6.6.

5. The method according to any one of claims 1 to 3, wherein whey is used as the milk material, and the pH of the protein-free liquid is adjusted to 3.6 to 5.5.

6. A sialyllactose material obtainable by a process comprising removing proteins from a milk material to obtain a protein-free liquid, adjusting a pH of the protein-free liquid to obtain a pH-adjusted liquid, and concentrating the pH-adjusted liquid by an ultrafiltration (UF) membrane.

7. A processed food comprising the sialyllactose material according to claim 6.

8. A processed food comprising 0.1 to 90 g of the sialyllactose material according to claim 6 per 100 g of the processed food.

9. A formula milk comprising the sialyllactose material according to claim 6.

10. A formula milk comprising 0.1 to 30 g of the sialyllactose material according to claim 6 per 100 g of the formula milk.

## INTERNATIONAL SEARCH REPORT

| International application No. |
| --- |
| PCT/JP2010/066669 |

**A. CLASSIFICATION OF SUBJECT MATTER**
*C07H7/027*(2006.01)i, *A23C9/142*(2006.01)i, *A23C9/152*(2006.01)i, *A23L1/30* (2006.01)i, *B01D61/14*(2006.01)i

According to International Patent Classification (IPC) or to both national classification and IPC

**B. FIELDS SEARCHED**

Minimum documentation searched (classification system followed by classification symbols)
C07H7/027, A23C9/142, A23C9/152, A23L1/30, B01D61/14

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2010 |
| Kokai Jitsuyo Shinan Koho | 1971-2010 | Toroku Jitsuyo Shinan Koho | 1994-2010 |

Electronic data base consulted during the international search (name of data base and, where practicable, search terms used)
CAplus/REGISTRY(STN), JSTPlus/JMEDPlus/JST7580(JDreamII)

**C. DOCUMENTS CONSIDERED TO BE RELEVANT**

| Category* | Citation of document, with indication, where appropriate, of the relevant passages | Relevant to claim No. |
| --- | --- | --- |
| X/Y | JP 4-316450 A (Snow Brand Milk Products Co., Ltd.), 06 November 1992 (06.11.1992), entire text; particularly, claims; paragraphs [0002], [0007] to [0009]; examples 4 to 7 & US 5270462 A | 1,5-10/1-10 |
| X/Y | JP 1-168693 A (Snow Brand Milk Products Co., Ltd.), 04 July 1989 (04.07.1989), entire text; particularly, claims; page 1, lower left column, 2nd line from the bottom to lower right column, line 10; page 2, lower left column, line 12 to page 3, upper left column, line 7; examples 1, 2 (Family: none) | 1,2,4-10/ 1-10 |

[X] Further documents are listed in the continuation of Box C.  [ ] See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 12 October, 2010 (12.10.10) | 26 October, 2010 (26.10.10) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2010/066669

C (Continuation).   DOCUMENTS CONSIDERED TO BE RELEVANT

| Category* | Citation of document, with indication, where appropriate, of the relevant passages | Relevant to claim No. |
|---|---|---|
| X/Y | WO 2007/051475 A1  (Arla foods amba), 10 May 2007 (10.05.2007), entire text; particularly, claims; examples & JP 2009-514511 A      & US 2007/0104843 A1 & EP 1951060 A1 | 6-10/1-10 |
| Y | WO 1999/08511 A1  (Neose Technologies Inc.), 25 February 1999 (25.02.1999), entire text; particularly, page 51, lines 24 to 31 & JP 2001-514865 A        & US 6323008 B1 & US 2004/0185146 A1      & US 6706497 B2 & EP 1003366 A1 | 3 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006117597 A
- JP H08252403 A
- JP H11180993 A
- JP 2003246800 A

**Non-patent literature cited in the description**

- *Shokuhin To Kaihatsu,* vol. 30, 10-13